# EUROPEAN PATENT SPECIFICATION

(11) **EP 1 187 573 B1**
(45) Date of publication and mention of the grant of the patent: **15.03.2006**
(21) Application number: 00937079.2
(22) Date of filing: 08.06.2000
(51) Int. Cl.: A61D 7/04, A61M 16/00

(54) **VENTILATION SYSTEM**
BEATMUNGSSYSTEM
SYSTEME DE VENTILATION

(30) Priority: 08.06.1999 GB 9913321; 08.06.1999 US 138057 P
(43) Date of publication of application: 20.03.2002
(73) Proprietor: Amersham Health AS, 0485 Oslo (NO); Johnson Controls, Inc., Alpharetta, GA 30005-4176 (US)
(72) Inventor: HARLING, Earl, Birdsboro, PA 19508 (US); ZAPPACOSTA, Nicholas, A., Norwood, PA 19074 (US); BATEMAN, Walt, E., Schwenksville, PA 19473 (US); ORLOWSKI, Robert, Schwenksville, PA 19473 (US); BYAR, Ann, A., Newtown Square, PA 19073 (US); TOBIN, Gary, F., West Chester, PA 19382 (US)
(74) Representative: Rollins, Anthony John
(86) International application number: PCT/GB2000/002226
(87) International publication number: WO 2000/074593

(56) References cited:
- EP-A- 0 300 110
- US-A- 4 053 604
- US-A- 4 332 244
- US-A- 4 582 055
- US-A- 4 949 714
- US-A- 5 099 792
- US-A- 5 297 502

## Description

The present invention relates to a ventilation system for use in a surgery suite.

A ventilation system as defined in the preamble of claim 1 is disclosed in US-A- 4 332 244.

Medical research frequently necessitates surgical procedures to be carried out on animals. One common form of surgical procedure is the cannulation of the jugular vein of rats and the insertion of telemetry devices. These procedures need to be carried out at least once a month by a researcher, and can last between one and four hours, depending on the number of rats requiring surgery and on any complications (such as equipment failure) which may arise during surgery.

The rats are anaesthetized during surgery, normally with a volatile liquid anaesthetic, optionally with a gaseous anaesthetic such as nitrous oxide. Suitable volatile liquid anaesthetics, which are non-explosive and non-flammable, include halothane (fluothane or 1-bromo-1-chloro-2,2,2-trifluoroethane), methoxyflurane (metofane or 2,2-dichloro-1,1-difluoroethyl methyl ether), and isoflurane (1-chloro-2,2,2-trifluoroethyl difluoromethyl ether). A preferred anaesthetic is isoflurane.

Clearly, it is desirable to reduce as far as possible the exposure of the persons carrying out the surgical procedures to the anaesthetic. Of course, one reason for this is to prevent drowsiness or the like in the person carrying out the procedure. However, exposure to large concentrations of isoflurane in particular can cause liver damage, decreased reproductive performance, and teratogenic effects (effects to an unborn embryo or foetus) such as fetotoxicity and cleft palate. The US National Institute for Occupational Safety and Health (NIOSH) sets a recommended maximum exposure level of 2 parts per million of waste anaesthetic per hour, although it will be appreciated that isoflurane levels must greatly exceed this recommended level to produce the adverse biological effects listed above.

As mentioned above, isoflurane is a volatile liquid anaesthetic, and so it must be vaporized using a vaporizer prior to its use on animals as an anaesthetic. However, anaesthetic systems using vaporizers are normally designed for use with humans or large animals. The system must therefore be modified in order to allow it to be used on small animals such as rats.

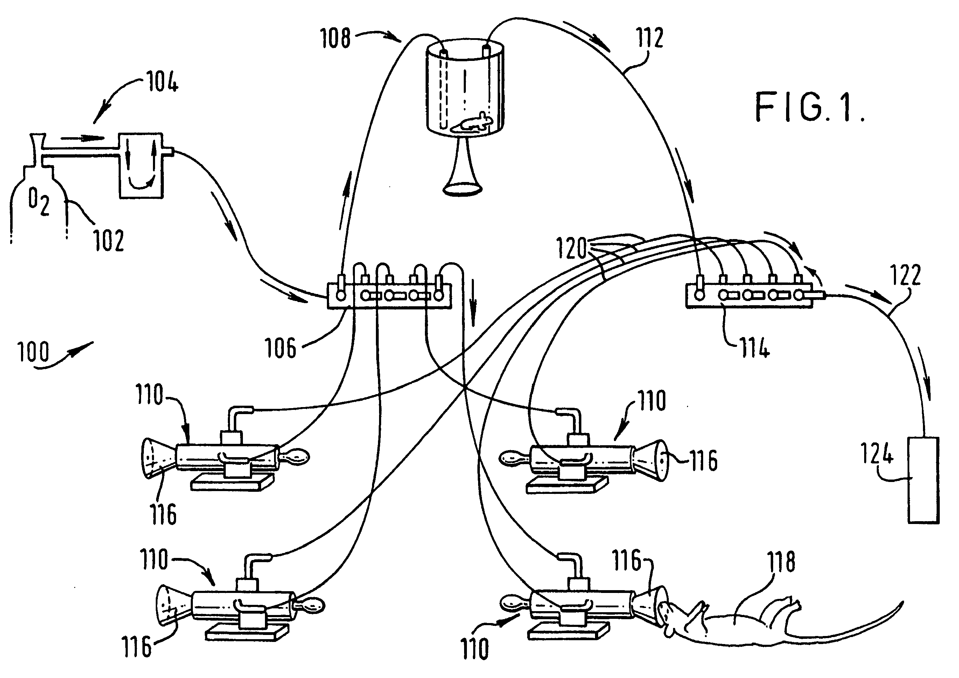

A known small animal surgery suite 100 allowing the use of isoflurane with rats is shown schematically in Figure 1. Oxygen is supplied at a rate of 300 ml to 1 1 per minute from a cylinder 102 and mixed with vaporized isoflurane in an anaesthesia machine, indicated at 104, and the mixture of oxygen and isoflurane is supplied to a valve box 106. From the valve box 106 the mixture flows to an induction chamber 108 and a number of breathing stations 110. Typically, the concentration of anaesthetic in the induction chamber, called the "induction concentration", is between about 10,000 - 50,000 parts per million (1% - 5%), and the in-line concentration of anaesthetic delivered to the breathing stations, called the "maintenance concentration", is between 15,000 - 35,000 parts per million. Tables 1 to 3 show recommended induction and maintenance concentrations of halothane, methoxyflurane and isoflurane when used with various animal species.

The induction chamber 108, in which the rats are initially anaesthetized, comprises an inverted bell jar with a lid through which two tubes pass. Anaesthetic mixture flows into the bell jar through one of the tubes, which extends to close to the base of the bell jar. The other tube, which functions as a return tube 112 for the anaesthetic mixture and exhaled gases, leads from the inside of the induction chamber 108 to a second valve box 114.

Each of the breathing stations 110, where the surgical procedure is carried out, includes a rubber fitting 116 with a hole cut into it. Once the rat 118 is anaesthetized, it is removed from the induction chamber and taken to the breathing station, where its nose is inserted into the hole in the rubber fitting 116. The anaesthetic is supplied to the breathing station 110 to ensure that the rat 118 is continuously sedated during the surgical procedure.

Return tubes 120 for the anaesthetic and exhaled gases lead from each of the breathing stations 110 to the second valve box 114, and a further pipe 122 leads from the valve box 114 to a charcoal filter 124, such as the F-air™ canister manufactured by Beckford, Inc. of Wales Center, New York, USA. The charcoal, of course, serves to remove organic vapours from the air passing through it, and must be replaced when saturated.

In practice, the induction chamber 108 and the breathing stations 110 are arranged along a table, and a general exhaust is positioned above the table. In addition, the room containing the surgery suite is normally under positive pressure to promote the exhaust of anaesthetic from the room. The air in the room is routinely exchanged at least eight times per hour while surgery is being performed. However, this prior art surgery suite may be inadequate in terms of the levels of anaesthetic released into the air.

To decrease the levels of anaesthetic released, a vacuum pump or similar device can be installed to suck anaesthetic into the return tubes and through the valve box into the charcoal filter. However, this can itself cause further difficulties, in that the vacuum pump may remove anaesthetic from the breathing stations before the anaesthetic has reached the animal being operated on. This can reduce the amount of anaesthetic reaching the animal to such a level that the animal can wake up before surgery is completed. This is obviously highly undesirable, not least from the viewpoint of causing unnecessary suffering to the animal.

According to a first aspect of the invention, there is provided a ventilation system for reducing the amount of anaesthetic released from an anaesthetic administration station into a surgery suite as defined in claim 1.

The exhaust is associated with means for entraining gas into the inlet. For example, the exhaust can be connected to a main fan, positioned elsewhere in the building housing the surgery suite. Alternatively, a fan dedicated to the exhaust, which may for example discharge through a window of the surgery suite, may be provided.

Since the inlet is positioned adjacent to the area where anaesthetic is released, a considerable portion of any released anaesthetic can be entrained into the inlet. The anaesthetic is thus captured and led to the exhaust before it can diffuse away from the area where it is released. This is distinct from the prior art in which released anaesthetic is allowed to pass from its area of release through the surgery suite to an exhaust.

In general, the anaesthetic administration station will be provided with a supply path, through which anaesthetic is supplied to the anaesthetic administration station, and a return path, through which waste anaesthetic and any gases exhaled by the animal being anaesthetized are removed from the anaesthetic administration station. It will be appreciated that the inlet and conduit of the system of the invention are provided in addition to the supply and return paths.

Where there are a plurality of areas of anaesthetic release (for example, more than one anaesthetic administration station), it is preferred that an inlet is provided adjacent to each area, to maximize the amount of anaesthetic which is entrained into the ventilation system.

The conduit leading from the inlet to the exhaust can take any suitable form. Of course, if there is a single area where anaesthetic is released, then a single pipe will suffice. However, if there are a plurality of areas, and a corresponding plurality of inlets, then each inlet may be joined to the exhaust. This can be done by providing a separate pipe extending from each inlet to the exhaust; however, it is preferred that the conduit comprises a main pipe connected at one end to the exhaust, and a plurality of branch pipes, each branch pipe connecting an inlet to said main pipe. This helps to reduce the overall size of the conduit.

Preferably, each branch pipe includes a valve for regulating flow in the branch pipe. The branch pipes can then be closed off, to prevent flow in them. This is useful if, for example, anaesthetic is only being released from some of the areas provided with inlets. The branch pipes whose inlets lead from these areas can then be closed off, allowing the entraining means to be concentrated on entraining into the inlets near where anaesthetic is being released.

As mentioned above, any suitable means can be used to entrain anaesthetic into the ventilation system. A flow of air may be drawn into the inlet, such air entraining released anaesthetic. The exhaust may be maintained at a lower pressure than the inlet, the pressure difference then serving to create an airflow and thus entrain the anaesthetic. In a preferred embodiment, the system comprises means for entraining anaesthetic in the form of a fan disposed in the region of said exhaust.

The ventilation system is particularly applicable for use in a small animal surgery suite, where the anaesthetic administration station is an induction chamber, where animals are initially anaesthetized.

Induction chambers used in small animal surgery suites normally take the form of bell jars with removable lids, as discussed above with reference to the prior art. In order for animals to be introduced into or removed from the induction chamber, the lid must be removed and then replaced. During the period when the lid is not on the chamber, it is possible for some of the relatively volatile anaesthetic to escape therefrom. Thus, it is preferred for the inlet to be provided above the induction chamber. In this way, only the anaesthetic escaping out of the chamber (for example when the lid is removed to insert an animal) will be entrained into the inlet; most of the anaesthetic will remain in the chamber, where it is required to anaesthetize the animals.

In a preferred form, the inlet is in the form of an inverted funnel. By drawing air into the inverted funnel, any escaping anaesthetic may be entrained into the ventilation system.

In an alternative form, the induction chamber comprises a plurality of compartments, including a first compartment where animals are initially anaesthetized and having means for the supply and removal of anaesthetic, and a second compartment connected to said inlet, the compartments being arranged such that anaesthetic escaping from the first compartment passes into the second compartment and thence to the inlet. Forming the induction chamber from a plurality of compartments in this way further reduces the amount of anaesthetic escaping into the general atmosphere, as any anaesthetic which does escape from the first chamber (where the animals are initially anaesthetized) must also escape from the second chamber in order to enter the general atmosphere.

The first and second compartments may be permanently connected. However, there is then a leakage path for anaesthetic direct from the first compartment to the second compartment. Thus, it is preferred that the first and second compartments are joined by a selectively closeable passage. The passage can be opened and closed to allow animals to be introduced into or removed from the first compartment.

It is further preferred that the inlet is at the top of the second compartment, and a lower region of the second compartment is provided with at least one ventilation hole for the intake of air. The exhaust system then produces a flow of air in the second compartment which entrains any anaesthetic escaping from the first compartment into the second compartment. Arranging the inlet at the top of the second compartment simplifies connection of the inlet to the exhaust system as a whole.

The ventilation system can also be applied to a small animal surgery suite where the anaesthetic administration station comprises at least one breathing station where surgery is carried out on an animal.

It is of course necessary to ensure that the animal remains anaesthetized at the breathing station, and this is done by supplying anaesthetic continuously to the animal undergoing surgery. It is possible for the anaesthetic to leak at the breathing station, and this is particularly undesirable, as the person carrying out the surgery is likely to have his or her face close to the animal, and thus can easily inhale the released anaesthetic.

In a preferred form, the or each breathing station includes an orifice for insertion of an animal's nose, the inlet being provided next to the orifice. Any anaesthetic leaking between the orifice and the animal's nose will then be substantially entrained into the ventilation system.

The inlet may be defined at an end of a length of tubing. In a particularly preferred form, the length of tubing is flexible. This allows the person carrying out surgery to move the tubing, and optionally the breathing station itself, as necessary. The length of tubing may form a branch pipe, as mentioned above, or it may be connected to such a branch pipe.

In a further preferred form, the inlet is formed as an annulus surrounding the orifice. This reduces the maximum distance which anaesthetic must travel in order to enter the inlet.

The invention also extends to a method of installing a system for reducing the amount of anaesthetic released from an anaeshetic administration station into a surgery suite as defined in claim 17.

In one form, the surgery suite is a small animal surgery suite, and the anaesthetic administration station is an induction chamber where animals are initially anaesthetized. It is then preferable for the method to further comprise positioning the inlet above the induction chamber.

In a further form, the surgery suite is a small animal surgery suite, and the anaesthetic administration station comprises at least one breathing station where surgery is carried out on an animal. Preferably the or each breathing station comprises an orifice for insertion of an animal's nose, the method further comprising positioning the inlet next to the orifice.

Preferred embodiments of the invention will now be described by way of example only and with reference to the accompanying drawings, in which:
Figure 1 shows a prior art ventilation system as described above;
Figure 2 is a schematic view of a preferred embodiment of the ventilation system of the invention;
Figure 3 is shows an induction chamber for use with the embodiment of Figure 2;
Figure 4 shows an alternative arrangement for the induction chamber;
Figure 5 shows a breathing station for use with the embodiment of Figure 2; and
Figure 6 shows an alternative arrangement for the breathing station.

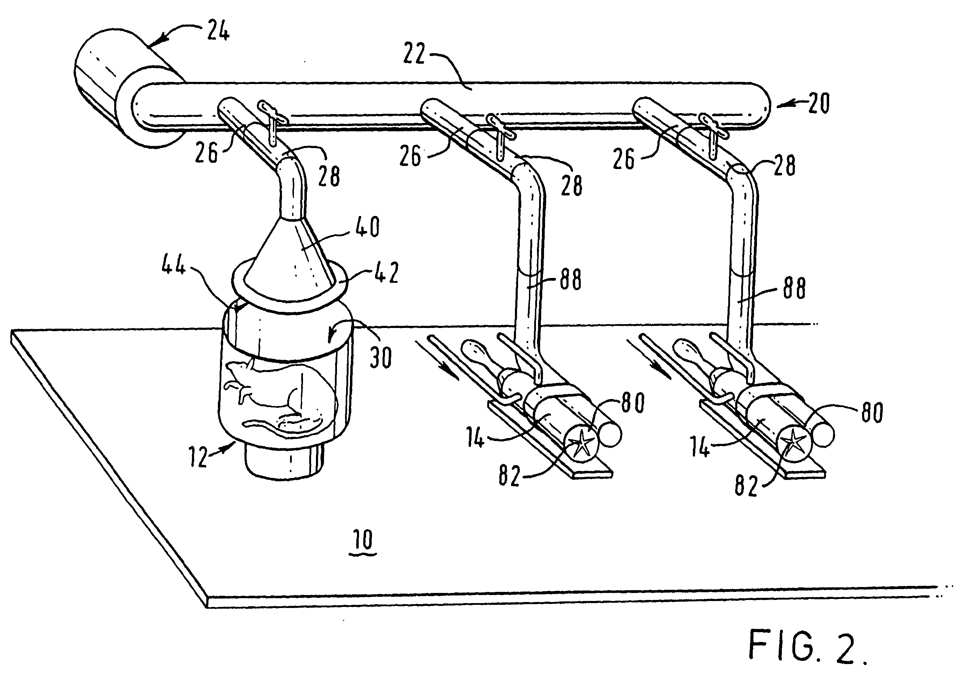

As shown in Figure 2, a preferred embodiment of the ventilation system includes an induction chamber 12 and a plurality of (in this case, two) breathing stations 14 arranged on a table 10, in a similar manner to the prior art system described above with reference to Figure 1. Of course, more or fewer breathing stations can be provided, depending on the requirements of the surgery suite.

The ventilation system 20 also includes means specifically designed to reduce the amount of anaesthetic escaping into the surgery suite. These means take the form of a main pipe 22, which is connected to an exhaust fan (indicated schematically at 24). A number of smaller pipes 26 branch from the main pipe 22 and lead to the induction chamber 12 and the breathing stations 14. When the exhaust fan is on, air from near the ends of the smaller branch pipes 26 is entrained into them, and thence into the main pipe 22 and the exhaust fan. The pipes are preferably formed from PVC, although any other suitable material can be used.

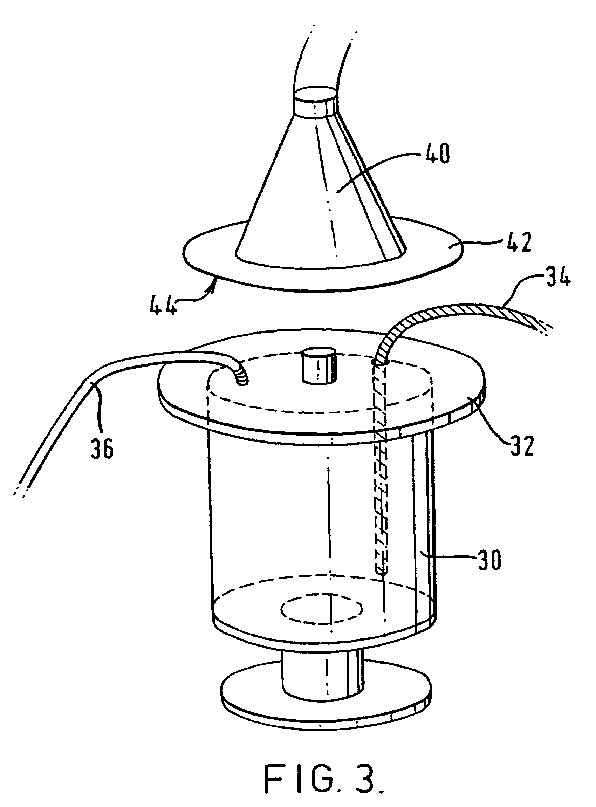

Turning now to the induction chamber, this is shown in more detail in Figure 3. As can be seen, the chamber 12 is in the form of an inverted bell jar 30 with a lid 32. Passing through the lid are two pipes 34 and 36. The first pipe 34 extends almost to the base of the bell jar 30, and is used for supplying anaesthetic. The second pipe 36, which terminates near to the lower surface of the lid 32, transports anaesthetic and exhaled gases away from the induction chamber 12 to a valve box and charcoal filter, as in the prior art. The pipes 34, 36 are sealed into the lid 32, and the lid 32 seals against the bell jar 30, such that there is no measurable leakage (measured using a MIRan portable infrared spectrophotometer) from the chamber 12 when the lid is closed.

Positioned above the bell jar is an inverted funnel 40, in the form of a flanged cone, which is connected to the exhaust. The diameter of the flange 42 substantially matches that of the bell jar. The lower end of the cone 44 is positioned a small distance above the top of the bell jar 30. Preferred measurements are a diameter for the flange of around 230 mm (9 inches), and a distance between the top of the bell jar and the bottom of the cone of around 25 mm (1 inch). Any anaesthetic which escapes out of the top of the bell jar 30 when the lid 32 is removed is entrained in a flow of air and carried to the exhaust.

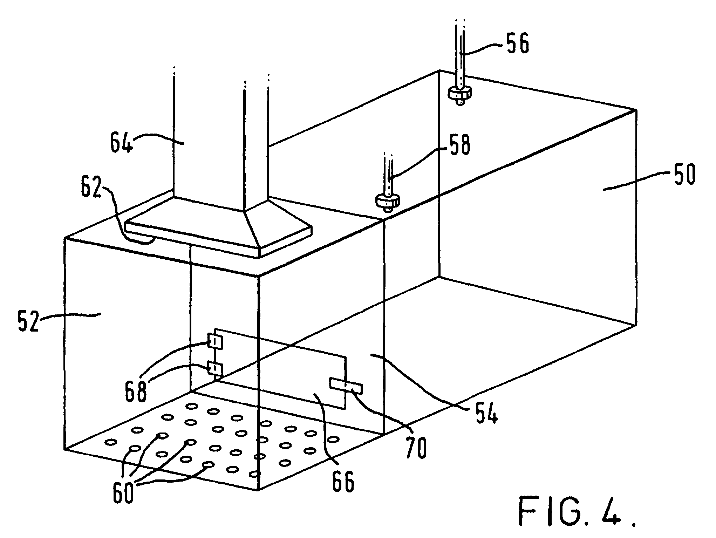

An alternative induction chamber is shown in Figure 4. Here, the induction chamber is formed from two compartments 50, 52 with a common wall 54. The compartments are shown as box-shaped, but can of course take any preferred shape. Preferably the compartments are formed from polymethyl methacrylate (Plexiglas or Perspex).

One of the compartments 50 has two pipes 56, 58 entering through its roof. The pipes are sealed into the roof so that there is no leakage at the points where they enter the compartment 50. These pipes 56, 58 are used to supply and remove anaesthetic, as in the first embodiment of the induction chamber. It is in this first compartment 50 that the animals are initially anaesthetized.

The second compartment 52 has a number of ventilation holes 60 formed in its bottom wall, and the compartment 52 is positioned such that air is free to flow upwardly through these holes 60. An opening 62 in the top wall of the second compartment 52 is connected to the exhaust system 64. When the exhaust system is operating, it sucks air through the ventilation holes 60 into the exhaust, so that there is a flow of air upwards, through the ventilation holes and into the exhaust, with an air evacuation speed of 0.48 m/s (94 feet per minute) in the centre of the opening 62 and 0.11 m/s (22 feet per minute) at the centre of the front face of the second compartment 52.

The front face of the second compartment is formed with a door (not shown), to allow access to the inside of the induction chamber. A second door 66 is formed in the common wall 54, attached to the common wall by hinges 68. Means (schematically indicated at 70) are provided for latching the door 66 closed. When closed, the door 66 seals the two compartments 50, 52 from each other, to prevent anaesthetic from leaking from the first compartment 50. Any anaesthetic which does leak between the compartments 50, 52 when the door 66 is closed is entrained into the upward air flow in the second compartment 52 and drawn into the exhaust. Similarly, when the door 66 between the compartments is opened to allow animals to be introduced into and removed from the first compartment 50, anaesthetic escaping through the open door 66 is drawn into the air flow and vented. This alternative arrangement thus further reduces the amount of anaesthetic escaping into the surgery suite, by ensuring that any anaesthetic escaping from the area where the animals are initially anaesthetized is immediately entrained into air flowing towards the exhaust. In addition, when the door in the front of the second compartment is open, air is drawn into the second compartment, and so the direction of air flow at the front of the second compartment, where a person handling an animal will be situated, is away from that person. Thus, the chance of anaesthetic being inhaled by that person is further reduced.

The size of the compartments 50, 52 will of course vary according to the size of the animals to be anaesthetized. However, a first compartment 50 having a height of around 228 mm (9 inches), a width of around 228 mm (9 inches) and a depth of around 241 mm (9.5 inches), a door 66 having a height and a width of around 178 mm (7 inches), and an exhaust intake in the top wall of the second compartment having a width of around 168 mm (6.625 inches) and a depth of around 89 mm (3.5 inches) have proved satisfactory for the anaesthetization of small rodents such as rats and mice.

Further, although the ventilation holes 60 have been shown in the bottom face of the second compartment 52, it will be appreciated that the holes can be positioned anywhere in the second compartment, as long as air flowing from the holes to the exhaust entrains the anaesthetic escaping from the first compartment 50.

An arrangement using inverted funnels, as in the first version of the induction chamber, can be used at the breathing stations 14. However, this is undesirable for a number of reasons. Firstly, the presence of the inverted funnel above the breathing station can obstruct the vision or movements of the person carrying out the surgical procedure. Secondly, the funnel only captures anaesthetic after it has entered the breathing zone of the person carrying out the procedure, and thus there is a chance that the person will inhale the anaesthetic. Since small animal surgery is exacting work, it is desirable to avoid these problems.

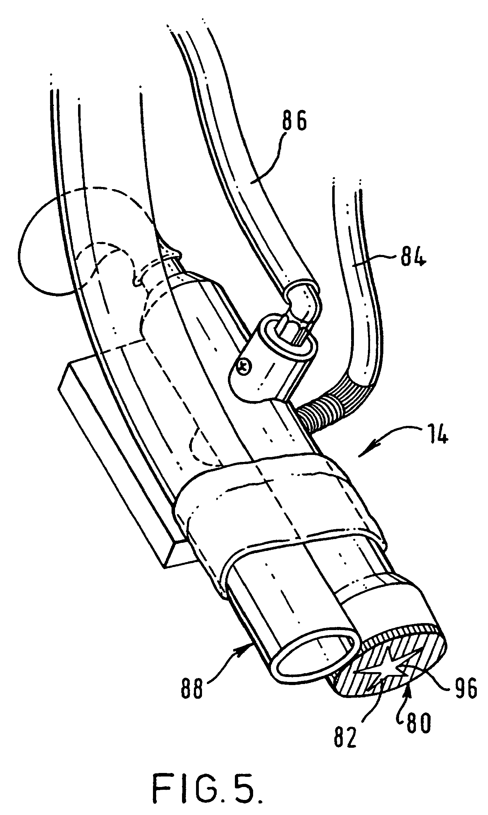

A preferred arrangement for reducing the amount of anaesthetic escaping from the breathing station is shown in more detail in Figure 5. The rubber fitting is replaced by an elastic diaphragm 80. The diaphragm is formed from relatively thick rubber or the like, and one or more slits 82 are formed in the diaphragm to allow insertion of the nose of a rat or similar animal. Supply and return tubes for the anaesthetic are shown at 84 and 86.

Further, rather than capturing the anaesthetic in an inverted funnel, a flexible tube 88 is attached to the breathing station 20 such that its mouth is level with the diaphragm 80, and the other end of the tube 88 is connected to one of the branch pipes 26. Rather than being level, it is also possible for the mouth of the tube to be offset back from the diaphragm by up to 6 mm (0.25 inches), and the tube may also project slightly forward of the diaphragm, as long as it does not interfere with the surgical procedure. The flexible tube 88 may be made from Tygon™, or any other suitable material. Anaesthetic leaking from the diaphragm 80 around the nose of the rat is captured by the air flowing into the tube 88 and conveyed away from the breathing station 14 before the person carrying out the surgical procedure can inhale it.

It will also be appreciated that it is only the anaesthetic which escapes from the breathing station which is entrained into the flexible tube. Most of the anaesthetic remains in the breathing station until it leaves by the return tube. The risk of the animal being operated on waking up during surgery as a result of the anaesthetic being removed from the breathing station before it is inhaled is much reduced.

To test the effectiveness of the system in preventing the escape of unwanted anaesthetic, and in particular to check that the mouth of the tube is positioned to allow escaping anaesthetic to be scavenged, the so-called "visual smoke test" is employed. In this test, smoke from a self-contained smoke source, such as a pipette containing burning material, is blown into the breathing station. It is then easy to see whether the smoke is entrained into the tube or not. If not, then it is necessary to change the position of the mouth of the tube until sufficient entrainment is achieved. This test is well known in the field as a standard way of detecting air flow.

In the preferred embodiment, the main pipe 22 has an inner diameter of around 38 mm (1.5 inches), and the smaller branch pipes 26 and the flexible tubing used to capture escaping anaesthetic at the breathing stations have internal diameters of around 19 mm (0.75 inches). The branch pipes 26 leading from the breathing stations are provided with 13 mm (0.5 inches) solvent weld PVC ball valves 28 at their junctions with the main pipe 22, and the branch pipe 26 leading from the induction chamber is provided with a similar 19 mm (0.75 inches) ball valve at its junction with the main pipe 22, allowing the flow in the pipes to be regulated. The main pipe is connected to an exhaust fan in the roof of the building housing the small animal surgery suite, which fan has a capacity of 18.8 m³/s (40,000 cubic feet per minute). When all of the valves 28 are open and the exhaust fan is on, air flows in the induction chamber branch pipe at a speed of around 0.33 to 0.36 m/s (65 to 70 feet per minute), and in the breathing station branch pipes at a speed of around 0.23 to 0.25 m/s (45 to 50 feet per minute).

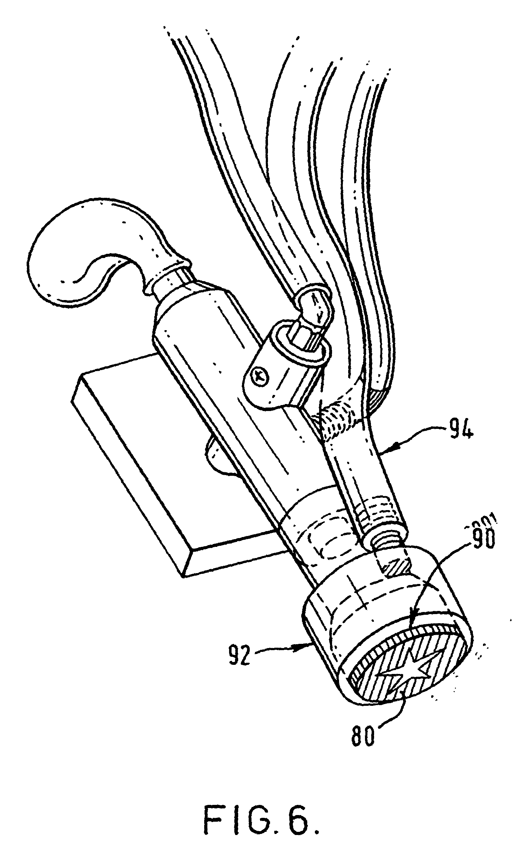

An alternative arrangement for preventing escape of anaesthetic at the breathing station is shown in Figure 6. In this arrangement, escaping anaesthetic is entrained into an annular gap 90 between the diaphragm 80 and a collar 92 which surrounds the end of the breathing station. The rear of the collar 92 is sealed to the breathing station 14 to prevent air being sucked in between the body of the breathing station and the rear of the collar. The collar is connected to one end of a length of Tygon™ tubing 94, whose internal diameter is 13 mm (0.5 inches) and whose external diameter is 19 mm (0.75 inches), the other end of which is connected to the branch pipe as in the previous arrangement.

The alternative arrangement has proved more effective than the previous one (as shown in Figure 5), where the tubing lies alongside the breathing station, in tests using the visual smoke test discussed above. This is because any anaesthetic escaping from the diaphragm is relatively close to the annular gap, irrespective of where on the diaphragm it escapes. This can be contrasted with the previous arrangement, where anaesthetic escaping from the edge of the diaphragm distant from the mouth of the tubing (as indicated by the reference numeral 96 in Figure 5) would have to be drawn across the entire diaphragm and around the anaesthetized animal before entering the tubing.

Tests carried out using the second arrangement showed an average speed of air flow across the diaphragm of around 1.85 m/s (360 - 370 feet/minute). Observation of the sedated animals showed that an air speed of this magnitude did not interfere with delivery of the anaesthetic to the animals. It is preferred for the air speed across the face to be between 0.25 and 2.03 m/s (50 and 400 feet/minute), and more preferably between 1.78 and 1.90 m/s (350 and 375 feet/minute), as air speeds of this nature minimize occupational exposure to waste anaesthetic while also minimizing interference with the anaesthetic delivery system.

In trials of the embodiment shown in Figures 5 and 6, the amount of isoflurane in the air inbreathing zone of the small animal surgery suite is reduced to below 0.02 parts per million as measured by the VAPOR-TRAK™ anaesthetic vapour monitor #8531 manufactured by KEM Medical Products Corporation, 14 Engineers Lane, Farmingdale, New York 11735, USA. THE VAPOR-TRAK™ anaesthetic vapour monitor #8531 is analyzed using OSHA (Occupational Safety and Health Administration) Method #29 for Halothane and Enflurane, developed by the Organic Methods Evaluation Branch, OSHA Analytical Laboratory, Salt Lake City, Utah, USA. This compares with tests of the known ventilation system, in which the level of isoflurane was of the order of 10 or more parts per million. It will thus be appreciated that the ventilation system greatly reduces the amount of anaesthetic escaping.

Of course, it will be appreciated that the induction chambers as described with reference to Figures 3 and 4 need not be used in combination with the breathing stations as described with reference to Figures 5 and 6, but can be used in any situation where an induction chamber of this nature is required. Likewise, the breathing stations can also be used independently of the induction chambers.

Although the invention has been described in the context of preventing excessive escape of anaesthetic from a small animal surgery suite, it will be appreciated that the ventilation system can be applied to other situations where it is desired to reduce the amount of noxious vapours escaping into the atmosphere.

**TABLE 1 Recommended Concentrations Of Volatile Anaesthetics: Halothane (Induction time: 3-5 minutes)**

| SPECIES | INDUCTION CONCENTRATION | MAINTENANCE CONCENTRATION* |
|---|---|---|
| Guinea Pig | 3-5% | 0.75-1.0% |
| Hamster or Gerbil | 3-5% | To effect |
| Mouse | 3-5% | To effect |
| Rabbit | 3-5% | 0.5-1.5% |
| Rat | 3-5% | To effect |

| | | |
|---|---|---|
| * When nitrous oxide is used in 50% concentration, the concentration of halothane can often be reduced by 10%. | | |

**TABLE 2 Recommended Concentrations Of Volatile Anaesthetics: Methoxyflurane (Induction time: Approximately 10 minutes) Note: Used primarily for rodents in a bell jar under a fume hood**

| SPECIES | INDUCTION CONCENTRATION | MAINTENANCE CONCENTRATION* |
|---|---|---|
| Guinea Pig | 3-3.5% (20-30 min) | To effect |
| Hamster or Gerbil | 3% | To effect |
| Mouse | 3% | To effect |
| Rabbit | To effect | 0-5-2% |
| Rat | 3% | To effect |

| | | |
|---|---|---|
| * When nitrous oxide is used in 50% concentration, the concentration of methoxyflurane can often be reduced by 10%. | | |

**TABLE 3 Recommended Concentrations Of Volatile Anaesthetics: Isoflurane (Induction time: 3-5 minutes)**

| SPECIES | INDUCTION CONCENTRATION | MAINTENANCE CONCENTRATION* |
|---|---|---|
| Guinea Pig | 1-4% | 1.5-3% |
| Hamster or Gerbil | 1-4% | 1.5-3% |
| Mouse | 1-4% | 1.5-3% |
| Rabbit | 3-4% | 2-3.5% |
| Rat | 1-4% | 1.5-3% |

| | | |
|---|---|---|
| * When nitrous oxide is used in 50% concentration, the concentration of isoflurane can often be reduced by 10%. | | |
| Note - Ferrets can be anaesthetized with halothane, methoxyflurane or isoflurane; however, no definite values are available. Induction and maintenance to effect only and monitor closely. | | |

## Claims

1. A ventilation system [20] for reducing the amount of anaesthetic released from an anaesthetic administration station into a surgery suite comprising a return tube [36, 86] leading from either an induction chamber [12] or a breathing station [14], **characterised in that** the system further comprises at least one inlet [40,88] positioned adjacent to an area of anaesthetic release from the anaesthetic administration station and **in that** the inlet [40,88] and the return tube [36,86] are each connected to an exhaust for conducting the anaesthetic out from the suite.

2. A ventilation system [20] as claimed in claim 1, wherein there are a plurality of areas of anaesthetic release, an inlet [40, 88] being provided adjacent to each area.

3. A ventilation system [20] as claimed in claim 2, comprising a main pipe [22] connected at one end to the exhaust, and a plurality of branch pipes [26], each branch pipe [26] connecting an inlet [40, 88] to said main pipe [22].

4. A ventilation system [20] as claimed in claim 3, wherein each said branch pipe [26] includes a valve for regulating flow in said branch pipe.

5. A ventilation system [20] as claimed in claims 1 to 4, comprising means for entraining air in the form of a fan [24] disposed in the region of said exhaust.

6. A ventilation system [20] as claimed in claims 1 to 5, wherein said surgery suite is a small animal surgery suite, and said anaesthetic administration station is an induction chamber [12], where animals are initially anaesthetized.

7. A ventilation system [20] as claimed in claim 6, wherein the inlet [40] is provided above the induction chamber [12].

8. A ventilation system [20] as claimed in claim 7, wherein the inlet [40] is in the form of an inverted funnel connected to the conduit.

9. A ventilation system [20] as claimed in claim 8, wherein the induction chamber [12] comprises a plurality of compartments, including a first compartment [50] where animals are initially anaesthetized and having means for the supply and removal of anaesthetic, and a second compartment [52] connected to said inlet [40], the compartments being arranged such that anaesthetic escaping from the first compartment [50] passes into the second compartment [52] and thence to the inlet [40].

10. A ventilation system [20] as claimed in claim 9, wherein said first [50] and second [52] compartments are joined by a selectively closeable passage.

11. A ventilation system [20] as claimed in 9 or claim 10, wherein said inlet [40] is at the top of the second compartment [52], and a lower region of said second compartment is provided with at least one ventilation hole [60] for the intake of air.

12. A ventilation system [20] as claimed in any preceding claim, wherein said surgery suite is a small animal surgery suite, and said anaesthetic administration station comprises at least one breathing station [14] where surgery is carried out on the animal.

13. A ventilation system [20] as claimed in claim 12, wherein the or each breathing station [14] includes an orifice for insertion of an animal's nose, the inlet [88] being provided next to the orifice.

14. A ventilation system as claimed in claim 13, wherein the orifice for the insertion of an animal's nose comprises a diaphragm (80) comprising one or more slits (82) to allow insertion of the nose of an animal.

15. A ventilation system [20] as claimed in claim 13 or claim 14, wherein said inlet [88] is defined at an end of a length of tubing.

16. A ventilation system [20] as claimed in claim any one of claims 12 to 14, wherein said inlet [88] is formed as an annulus surrounding the orifice.

17. A method of installing the ventilation system [20] according to claims 1-16, the method comprising positioning at least one inlet [40,88] adjacent to an area of anaesthetic release from the anaesthetic administration station, and connecting the inlet [40, 88] to an exhaust by means of a conduit.

18. A method as claimed in claim 17, wherein said surgery suite is a small animal surgery suite, and said anaesthetic administration station is an induction chamber [12], where animals are initially anaesthetized.

19. A method as claimed in claim 17 or claim 18, wherein said surgery suite is a small animal surgery suite, and said anaesthetic administration station comprises at least one breathing station [14] where surgery is carried out on an animal.

## Patentansprüche

1. Belüftungssystem [20] zum Verringern der Menge von Anästhetikum, das aus einer Anästhetikum-Verabreichungsstation in einen Operationsraum freigesetzt wird, umfassend ein Rücklaufrohr [36, 86], das von entweder einer Ansaugkammer [12] oder einer Beatmungsstation [14] ableitet, **dadurch gekennzeichnet, dass** das System weiter mindestens einen Einlass [40, 88] umfasst, der benachbart zu einem Gebiet der Freisetzung des Anästhetikums aus der Anästhetikum-Verabreichungsstation positioniert ist, und dass der Einlass [40, 88] und das Rücklaufrohr [36, 86] jeweils mit einem Auslass zum Leiten des Anästhetikums aus dem Raum verbunden sind.

2. Belüftungssystem [20] nach Anspruch 1, bei dem es eine Mehrzahl von Gebieten der Freisetzung des Anästhetikums gibt, wobei ein Einlass [40, 88] benachbart zu jedem Gebiet vorgesehen ist.

3. Belüftungssystem [20] nach Anspruch 2, umfassend ein Hauptrohr [22], das an einem Ende mit dem Auslass verbunden ist, und eine Mehrzahl von Verzweigungsrohren [26], wobei jedes Verzweigungsrohr [26] einen Einlass [40, 88] mit dem Hauptrohr [22] verbindet.

4. Belüftungssystem [20] nach Anspruch 3, bei dem jedes Verzweigungsrohr [26] ein Ventil zum Regulieren des Flusses in dem Verzweigungsrohr umfasst.

5. Belüftungssystem [20] nach den Ansprüchen 1 bis 4, umfassend ein Mittel zum Einfangen von Luft in der Form eines Ventilators [24], der in dem Bereich des Auslasses angeordnet ist.

6. Belüftungssystem [20] nach den Ansprüchen 1 bis 5, bei dem der Operationsraum ein kleiner Tier-Operationsraum ist und die Anästhetikum-Verabreichungsstation eine Ansaugkammer [12] ist, in der Tiere anfänglich anästhesiert werden.

7. Belüftungssystem [20] nach Anspruch 6, bei dem Einlass [40] oberhalb der Ansaugkammer [12] vorgesehen ist.

8. Belüftungssystem [20] nach Anspruch 7, bei dem der Einlass [40] in der Form eines umgedrehten Trichters, der mit dem Abzug verbunden ist, vorliegt.

9. Belüftungssystem [20] nach Anspruch 8, bei dem die Ansaugkammer [12] eine Mehrzahl von Kabinen umfasst, umfassend eine erste Kabine [50], in der Tiere anfänglich anästhesiert werden und aufweisend ein Mittel für die Lieferung und Entfernung eines Anästhetikums, und eine zweite Kabine [52], verbunden mit dem Einlass [40], wobei die Kabinen derart angeordnet sind, dass das Anästhetikum, dass aus der ersten Kabine [50] ausströmt, in die zweite Kabine [52] und dann in den Einlass [40] läuft.

10. Belüftungssystem [20] nach Anspruch 9, bei dem die erste [50] und zweite [52] Abteilung durch eine selektiv verschliessbare Passage verbunden sind.

11. Belüftungssystem [20] nach Anspruch 9 oder 10, bei dem der Einlass [40] am oberen Ende der zweiten Kabine [52] vorliegt, und ein unterer Bereich der zweiten Kabine mit mindestens einem Belüftungsloch [60] zum Einlass von Luft versehen ist.

12. Belüftungssystem [20] nach einem der vorstehenden Ansprüche, bei dem der Operationsraum ein kleiner Tier-Operationsraum ist und die Anästhetikum-Verabreichungsstation mindestens eine Beatmungsstation [14] umfasst, in der eine Operation des Tieres ausgeführt wird.

13. Belüftungssystem [20] nach Anspruch 12, bei dem die oder jede Beatmungsstation [14] eine Öffnung zum Einsetzen der Nase eines Tieres umfasst, wobei der Einlass [88] in der Nähe der Öffnung vorgesehen ist.

14. Belüftungssystem nach Anspruch 13, bei dem die Öffnung zum Einsetzen der Nase eines Tieres ein Diaphragma [80] umfasst, das eine oder mehrere Schlitze [82] aufweist, um das Einsetzen der Nase eines Tieres zu ermöglichen.

15. Belüftungssystem [20] nach Anspruch 13 oder 14, bei dem der Einlass [88] an einem Ende einer Länge eines Schlauches definiert ist.

16. Belüftungssystem [20] nach einem der Ansprüche 12 bis 14, bei dem der Einlass [88] als ein Ringraum gebildet ist, der die Öffnung umgibt.

17. Verfahren zum Installieren des Belüftungssystems [20] nach den Ansprüchen 1 bis 16, wobei das Verfahren umfasst Positionieren mindestens eines Einlasses [40, 88] benachbart zu einem Gebiet der Freisetzung eines Anästhetikums aus der Anästhetikum-Verabreichungsstation, und Verbinden des Einlasses [40, 88] mit einem Auslass mittels eines Abzuges.

18. Verfahren nach Anspruch 17, bei dem der Operationsraum ein kleiner Tier-Operationsraum ist und die Anästhetikum-Verabreichungsstation eine Ansaugkammer [12] ist, in der Tiere anfänglich anästhesiert werden.

19. Verfahren nach Anspruch 17 oder 18, bei dem der Operätionsraum ein kleiner Tier-Operationsraum ist und die Anästhetikum-Verabreichungsstation mindestens eine Beatmungsstation [14] umfasst, an der eine Operation eines Tieres ausgeführt wird.

## Revendications

1. Système de ventilation [20] permettant de réduire la quantité d'anesthésiant émis par un poste d'administration d'anesthésiant dans une suite chirurgicale comprenant un tube de retour [36, 86] partant soit d'une chambre d'induction [12] soit d'un poste respiratoire [14], **caractérisé en ce que** le système comprend en outre au moins un orifice d'entrée [40, 88] disposé de manière contiguë à une zone d'émission d'anesthésiant provenant du poste d'administration d'anesthésiant et **en ce que** l'orifice d'entrée [40, 88] et le tube de retour [36, 86] sont chacun raccordés à un dispositif d'échappement permettant d'évacuer l'anesthésiant hors de la suite.

2. Système de ventilation [20] selon la revendication 1, dans lequel il est prévu une pluralité de zones d'émission d'anesthésiant, un orifice d'entrée [40, 88] étant prévu de manière contiguë à chaque zone.

3. Système de ventilation [20] selon la revendication 2, comprenant un tuyau principal [22] raccordé à une extrémité au dispositif d'échappement, et une pluralité de tuyaux de ramification [26], chaque tuyau de ramification [26] reliant un orifice d'entrée [40, 88] audit tuyau principal [22].

4. Système de ventilation [20] selon la revendication 3, dans lequel chacun desdits tuyaux de ramification [26] comprend une vanne servant à réguler le flux présent dans ledit tuyau de ramification.

5. Système de ventilation [20] selon l'une des revendications 1 à 4, comprenant un moyen permettant d'entraîner l'air sous la forme d'un ventilateur [24] disposé dans la zone dudit dispositif d'échappement.

6. Système de ventilation [20] selon l'une des revendications 1 à 5, dans lequel ladite suite chirurgicale est une suite chirurgicale pour petits animaux, et ledit poste d'administration d'anesthésiant est une chambre d'induction [12] dans laquelle les animaux sont tout d'abord anesthésiés.

7. Système de ventilation [20] selon la revendication 6, dans lequel l'orifice d'entrée [40] est prévu au-dessus de la chambre d'induction [12].

8. Système de ventilation [20] selon la revendication 7, dans lequel l'orifice d'entrée [40] a la forme d'un entonnoir inversé raccordé à la conduite.

9. Système de ventilation [20] selon la revendication 8, dans lequel la chambre d'induction [12] comprend une pluralité de compartiments, comportant un premier compartiment [50] dans lequel les animaux sont tout d'abord anesthésiés, et ayant un moyen permettant l'introduction et l'évacuation d'anesthésiant, et un second compartiment [52] raccordé audit orifice d'entrée [40], les compartiments étant agencés de telle manière que l'anesthésiant sortant du premier compartiment [50] passe dans le second compartiment [52] et, de là, dans l'orifice d'entrée [40].

10. Système de ventilation [20] selon la revendication 9, dans lequel ledit premier [50] et ledit second [52] compartiment sont raccordés par un passage pouvant être fermé de manière sélective.

11. Système de ventilation [20] selon la revendication 9 ou la revendication 10, dans lequel ledit orifice d'entrée [40] se situe en haut du second compartiment [52], et une zone plus basse dudit second compartiment est munie d'au moins un trou de ventilation [60] permettant un apport d'air.

12. Système de ventilation [20] selon l'une quelconque des revendications précédentes, dans lequel ladite suite chirurgicale est une suite chirurgicale pour petits animaux, et ledit poste d'administration d'anesthésiant comprend au moins un poste respiratoire [14] dans lequel les animaux subissent une chirurgie.

13. Système de ventilation [20] selon la revendication 12, dans lequel le ou chaque poste respiratoire [14] comprend un orifice à insérer dans le museau d'un animal, l'orifice d'entrée [88] étant prévu proche de l'orifice.

14. Système de ventilation [20] selon la revendication 13, dans lequel l'orifice à insérer dans le museau d'un animal comprend un diaphragme [80] comprenant une ou plusieurs fentes [82] permettant l'insertion du museau de l'animal.

15. Système de ventilation [20] selon la revendication 13 ou la revendication 14, dans lequel ledit orifice d'entrée [88] est défini à une extrémité d'une longueur de tube.

16. Système de ventilation [20] selon l'une quelconque des revendications 12 à 14, dans lequel ledit orifice d'entrée [88] est formé comme un anneau entourant l'orifice.

17. Procédé d'installation d'un système de ventilation [20] selon l'une des revendications 1 à 16, le procédé comprenant la disposition d'au moins un orifice d'entrée [40, 88] de manière contiguë à une zone d'émission d'anesthésiant provenant du poste d'administration d'anesthésiant, et la connexion de l'orifice d'entrée [40, 88] à un dispositif d'échappement au moyen d'une conduite.

18. Procédé selon la revendication 17, dans lequel ladite suite chirurgicale consiste en une suite chirurgicale pour petits animaux, et ledit poste d'administration d'anesthésiant consiste en une chambre d'induction [12] dans laquelle les animaux sont tout d'abord anesthésiés.

19. Procédé selon la revendication 17 ou la revendication 18, dans lequel ladite suite chirurgicale est une suite chirurgicale pour petits animaux, et ledit poste d'administration d'anesthésiant comprend au moins un poste respiratoire [14] dans lequel les animaux subissent une chirurgie.
